Europäisches Patentamt

(19) European Patent Office

Office européen des brevets

(11) Publication number: **0 444 946 A2**

## (12) EUROPEAN PATENT APPLICATION

(21) Application number: **91301694.5**

(22) Date of filing: **01.03.91**

(51) Int. Cl.⁵: **G01N 30/78, G01N 33/28**

(30) Priority: **02.03.90 US 487579**

(43) Date of publication of application:
**04.09.91 Bulletin 91/36**

(84) Designated Contracting States:
**BE DE ES FR GB GR IT NL SE**

(71) Applicant: **EXXON RESEARCH AND ENGINEERING COMPANY**
**P.O.Box 390, 180 Park Avenue**
**Florham Park, New Jersey 07932 (US)**

(72) Inventor: **Schulz, Wolfgang Walter**
**29 Old Montain Road**
**Lebanon, New Jersey 08833 (US)**
Inventor: **Genowitz, Mark Wayne**
**20 Johanna Way**
**Phillipsburg, New Jersey 08865 (US)**

(74) Representative: **Somers, Harold Arnold et al**
**ESSO Engineering (Europe) Ltd. Patents & Licences Mailpoint 72 Esso House Ermyn Way**
**Leatherhead, Surrey KT22 8XE (GB)**

(54) **A method for quantitatively measuring saturates, olefins and aromatics in a composition.**

(57)   A method for quantitatively determining the amount of saturate, olefin and aromatic components in a composition or mixture is described. The method involves contacting a sample of a composition with a stationary phase; separating the saturates, olefins and aromatics loaded on the stationary phase by passing a supercritical fluid mobile phase through the stationary phase to obtain an eluent, wherein the amount of saturates, olefins and aromatics in the eluent varies over time and passing the eluent through an ultraviolet absorption (Uv) detector for irradiating the eluent with ultraviolet light at a wavelength capable of producing a uniform ultraviolet absorbance response for the olefins in the sample; measuring the ultraviolet absorbance of the olefins in the eluent; and passing the eluent through a mass detector (FID), capable of measuring the mass response to saturates, olefins and aromatics in the eluent and determining the quantitative amounts of saturates, olefins and aromatics in the composition from the detectors.

EP 0 444 946 A2

FIG. 1

# A METHOD FOR QUANTITATIVELY MEASURING SATURATES OLEFINS AND AROMATICS IN A COMPOSITION

## BACKGROUND OF THE INVENTION

### Field of the Invention

The present invention relates to an analytical method for measuring the amount of saturates, olefins and aromatics in a composition. More specifically, the invention is an analytical method for quantitatively determining the amount of saturate, olefin and aromatic components in a composition using supercritical fluid chromatography separation techniques in conjunction with an ultraviolet absorbance (Uv) detector and a flame ionization detector (FID).

### Background of the Disclosure

A fundamental goal of analytical chemistry is the identification and quantitative analysis of specific components or specific groups of components in a composition. Identification generally cannot proceed, however, until the components of the composition have been, to some extent, physically separated from the composition. One of the most widely used techniques for separation is chromatography which relies on the differing relative affinities each component in the composition will have for the stationary and mobile phases of the chromatography.

The stationary phase is typically a suitable particulate solid material in the form of a column and the mobile phase is typically a gas, liquid or supercritical fluid solvent. The mobile phase is continually passed through the column of solid stationary phase. Thereafter a small sample of the composition under investigation is passed through the column followed by the mobile phase alone. As previously mentioned, various components of the composition will have different affinities for the stationary phase and, therefore, will be retained at different regions along the length of the columnar stationary phase for different times periods. For some components, the affinity will be so slight that virtually no retention will be evident, while for others the affinity might be so great that the components are never recovered from the stationary phase. This may continue to be so even after considerable periods of time have elapsed and after the components are subjected to the eluting properties of the mobile phase being passed through the stationary phase.

Several techniques of chromatographic analysis exist these include: gas chromatography, gas liquid chromatography, gas solid chromatography, thin layer chromatography, liquid chromatography, high performance liquid chromatography and supercritical fluid chromatography. The present invention is directed to supercritical fluid chromatography (SFC). However, other chromatography separation techniques may be used provided they allow for suitable separation of the components, vis-a-vis their relative affinities for the mobile or stationary phases of the chromatography. Of the various chromatography techniques used to separate the components of mixtures each has its own limitations.

The technique used to identify the components of a composition, once they have been partially or completely separated by the chromatography, is defined herein as "detection". The various detectors include Uv, FID, refractive index, fluorescence and dielectric constant detectors. The choice of detector depends on the properties of the components since some may be inherently suitable for detection while other may not possess a suitable detectable property. For example, the component may not fluoresce or fluoresce with the degree or wavelength required for detection.

In order to complete the chromatography analysis, the chromatography separation column and detector are combined or linked together to form a single chromatography system that separates and identifies the separated components of the compostion so that the amount of each component may be determined.

A number of chromatography systems have been employed in the prior art. For example, H. Boer, P. Arkel and W. Boersma, "An Automatic PNA Analyzer for the Under 200°C Fraction Contained in A Higher Boiling Product" Chromatographia 13 (8): 500-12; 1980, reveals a gas chromatography combined with FID to determine the amount of paraffins, naphthenes, aromatics and olefins in kerosene gas-oil fractions. The chromatography employs multiple chromatograph columns of polar and non-polar stationary phases. The olefin content is indirectly determined by means of a special module used to theoretically calculate the olefin content.

J. V. Brunnock and L. A. Luke, "Rapid Separation by Carbon Number and Determination of Naphthene and Paraffin Content of Saturate Petroleum Distillates Up to 185°C", Analytical Chem. 40 (14): 2158-67; 1968, shows a gas solid chromatography that uses novel molecular sieve which permits separation of naphthene and paraffins from hydrocarbon feedstocks boiling up to 185°C. The novel sieves could also be used in gas liquid chromatography. An FID is used to determine the quality of separation for the various naphthene and paraffin components.

L. Schmidt, H. Danigel and H. Jungclas, "Combined Liquid Chromatography-Mass Spectrometry for Trace Analysis of Pharmaceuticals", Nuclear Instruments and Methods, Vol. 198, pp. 165-167 (1982) discloses combining liquid chromatography with a mass spectrometer detector for determining trace amounts of pharmaceuticals in biological fluids like blood and urine.

U.S. Patents 4,778,764 and 4,843,016, issued to Fine et al. teaches detecting the presence of predetermined compounds in a sample. The system comprises high pressure liquid chromatography column or gas chromatography column. The eluent from the column is injected into a combustion chamber where nitrogen, sulfur and carbon in the sample are converted into gaseous nitric oxide, sulfur dioxide and carbon dioxide respectively. Specific gas detectors are then used to detect the amounts of these components in the combustion product.

U.S. Patent 4,757,023, issued to Trestianu, et al. and U.S. Patent 3,169,389, issued to L. E. Green, Jr., et al., teach gas chromatography and FID as a means for separating mixtures of organic compounds and petroleum products by their boiling range.

U.S. Patents 4,802,986 and 4,826,603, issued to Hayes, Jr., et al., teaches high pressure liquid chromatography for analyzing crude oils and petroleum products for saturates, olefins and aromatics. Either single or dual chromatography columns are used. In the case of dual columns, the first column is packed with a porous silica packing material having a polar aminocyano bonded phase, while the second column is packed with a tetranitrofluorenimino material. The stationary phase consist of silica gel and a bonded phase comprising aromatic benzene sulfonic acid functional groups. A dielectric constant detector is used to quantitatively identify saturates, aromatics and olefins in the eluent. However, when dual chromatographic columns are used, the detector is calibrated to detect saturates, alkyl benzenes and aromatics only. On the other hand, when a single chromatography column is used, the saturate and aromatic components are first eluted from the olefin selective column in a forward flow direction while the olefins are retained on the stationary phase. Afterwards, the flow of material is rerouted, a process known as backflushing, so that the olefins, remaining on the stationary phase of the column, pass through the detector. Therefore, the chromatography system requires some knowledge of the elution rate for the saturate and aromatic components beforehand in order to activate the backflush operation at the proper time.

U.S. Patent 4,631,687, issued to Kowalski, et al., also teaches using two columns in liquid chromatography each selected to produce a qualitatively different type of separation. A number of different types of detectors may be used, which include optical and mass spectrometers. The detectors are multi-channel detectors, since single-channel detectors are not capable of identifying the components as in the case of optical detectors. These typically operate at a single wavelength and are not particularly unique identifiers of a chemical species. The multi-channel detector simultaneously measures the absorption of the components at several different wavelengths.

U.S. Patent 4,233,030, issued to Twitchett, et al., represents another example where liquid chromatography is used in conjunction with a Uv detector to detect a known or suspected constituent. The species is converted by irradiation to have enhanced light absorbance at a particular wavelength or to have reduced light absorbance at a particular wavelength. Therefore, the irradiation serves to increase the sensitivity and/or the selectivity of detector.

There has been recent interest in using supercritical fluids chromatography (SFC) for group separation since it offers the potential to conduct analyses that are too difficult to be performed with either gas, liquid or high pressure liquid chromatography. From a chromatography perspective, the low viscosity, high diffusivity and solvating power of supercritical fluids are the attractive features in SFC. The prior art has also sought to combine this separation technique with a number of detection methods.

An example of such combination is illustrated in E. Lundanes, B. Iversen and T. Greibrokk, "Group Separation of Petroleum Using Supercritical Fluids", Journal of Chromatography, Vol. 366, pp. 391-395 (1986). The article examines the possibility of using supercritical fluids such as carbon dioxide, nitrous oxide, sulfur hexafluoride and ammonia as the mobile phase for the group separation of gasoline and high boiling petroleum fractions. The stationary phase was made of silica and the detector employed was FID. When carbon dioxide was used as the supercritical fluid mobile phase, it was necessary to impregnate the silica stationary phase with silver nitrate to obtain complete separation of the saturates and aromatics. Group separation into saturates, aromatics and polar compounds of the high boiling petroleum fraction was obtained using a three column system with columnswitching and back-flushing.

H. E. Schwartz and R. G. Brownlee, "Hydrocarbon Group Analysis of Gasolines with Microbore Supercritical Fluid Chromatography and Flame Ionization Detection", Journal of Chromatography, Vol. 353, pp. 77-93 (1986), discusses the use of sulfur hexafluoride as a supercritical fluid mobile phase to affect the separation of paraffins and olefins in petroleum samples using an FID. The stationary phase was made of silica and care was taken to keep the sulfur hexafluoride as dry as possible. Although, silver loading of the silica columns was not necessary, the aromatics were eluted with temperature, flow and high pressure programming.

R. M. Campbell, N. M. Djordjevic, K. E. Markides and M. L. Lee, "Supercritical Fluid Chromatography Determination of Hydrocarbon Groups in Gasolines and Metal Distillate Fuels", Analytical Chemistry, Vol. 60, pp. 356-362 (1988), discusses supercritical fluid dual column chromatography to separate and determine the amounts of saturates, olefins and aromatics in gasolines and middle distillate fuels using a FID. The stationary phase in one column is a silica gel and is used to isolate and determine the aromatics. A second silver ion loaded silica gel column was used to separate the saturates from the olefins in aromatics. The olefins are then determined by difference or by using a column switching technique. The supercritical fluid is a mixture of carbon dioxide and sulfur hexafluoride.

Due to the limitations of these chromatography separations, it has also been proposed that dual detectors could be incorporated with supercritical fluid chromatography. One detector would serve as a universal detector, such as a FID and the second detector could serve as a specific detector, such as a Uv detector.

A dual detector chromatography system is discussed in D. J. Bornhop, S. Schmidt and N. L. Porter, "Use of Two Simultaneous Detectors in Capillary Supercritical Fluid Chromatography", Journal of Chromatography, Vol. 459, pp. 193-200 (1988). The article describes a flow splitter device that allows simultaneous detection with Uv and FID detectors in a capillary supercritical fluid chromatography system. The Uv detector was especially designed for capillary supercritical fluid chromatography. The supercritical fluid mobile phase is carbon dioxide and the stationary phase is a mixture of SB-methyl-50 and SB-methyl-100. The chromatography was designed to separate anthracene, pyrene, $C_{16}$ and $C_{24}$. The Uv detector was calibrated to detect anthracene and pyrene at wavelengths of 210-254 nanometers. There were at least three major compounds absorbing strongly at 254 nanometers not detected by the FID.

T. A. Norris and M. G. Rawdon, "Determination of Hydrocarbon Types in Petroleum Liquids by Supercritical Fluid Chromatography With Flame Ionization Detection", Analytical Chemistry, Vol. 56, pp. 1767-1769 (1984), investigated the possibility of using supercritical fluid chromatography with a silica stationary phase combined with a Uv detector operating at a wavelength of 190 nanometers and a FID. The Uv detector was used only to demonstrate whether there was complete seperation of olefins from aromatics, but was not used for the quantitation of separated components. The quantitation was restricted to measurements derived from the FID. Since the FID operates as a universal detector the measurement of saturates, olefins and aromatics required complete separation of these components. The separation of olefins from saturates was accomplished by using an additional column loaded with a silver nitrate stationary phase.

U.S. Patent 4,865,746, issued to Overfiled, teaches high pressure liquid chromatography used in conjunction with Uv and FID. The mixture is passed through a stationary phase and a mobile phase consisting of solvents of varying solubility. Optionally, the mobile phase could be a liquid, gas or a supercritical fluid. The Uv detector is operated at wavelengths of 200-400 nanometers. The aim of the anaylsis is to quantify the concentration of aromatic compounds in the mixture. The aromatic compounds are of differing ring numbers and substitutions all of which have differing ultraviolet light absorbance spectra.

The standard method used in the petroleum industry for measuring the quantitative amount of saturates, olefins and aromatics in a hydrocarbon composition is discussed in "Hydrocarbon Types in Liquid Petroleum Products by Fluorescence Indicator Adsorption", ASTM Test No. D1319. This method, also known as FIA, however, suffers from a number of drawbacks some being: (1) time consuming, (2) operator intensive, (3) low in accuracy, (4) the need to pretreat the sample by removing pentanes, (5) interference from colored samples, (6) limited applicability to hydrocarbon materials boiling at temperatures greater than 315°C and (7) an inability to measure low olefin contents.

The prior art attempts to separate the saturates, olefins and aromatics in a composition, unfortunately, complete separation of these components is the exception, rather than the rule. When chromatography separation is very poor or nonexistent, the analyst may not even be able to know that a separation problem exists, since two or more of the components may migrate down the stationary phase at the same rate and, thus, not be detected or be detected as a single component. Even when some degree of separation is achieved, the problem of identifying overlapped components is one that has plagued chromatography since its inception.

Finally, while all of the above methods strive to improve on the existing standard laboratory method used in the petroleum industry for quantitatively measuring the amount of saturates, olefins and aromatics in hydrocarbon compositions, many of them contain such a high level of instrumentation and complexity that it precludes their use for routine industrial applications requiring ease in use, speed and reliability.

It is therefore, an object of the present invention to provide an analytical method whereby the quantitative amount of saturates, olefins and aromatics in a composition are easily and quickly determined with greater reliability and eliminates the need for column switching and back-flushing.

EP 0 444 946 A2

It is a further object of the present invention to provide an analytical method that eliminates the need to separate the olefin and saturate components in a composition.

Other objects and advantages of the present invention will be apparent to those skilled in the art, particularly upon reading the examples and disclosures herein.

## SUMMARY OF THE INVENTION

The invention provides a method for quantitatively determining the amount of saturate, olefin and aromatic components in a composition or mixture. The method involves contacting a sample of the composition with a stationary phase for a time sufficient to load the saturates, olefins and aromatics onto the stationary phase; separating the saturates, olefins and aromatics loaded on the stationary phase by passing a mobile phase, under supercritical conditions, through the stationary phase in a sufficient amount and for a time sufficient to obtain an eluent, wherein the amount of saturates, olefins and aromatics in the eluent varies over time such that the amount of saturates and olefins in the eluent obtained over a first time interval is substantially higher than over a second time interval, being a time interval at least after the first time interval and the amount of aromatics in the eluent obtained over the second time interval is substantially higher than over the first time interval; passing the eluent through an ultraviolet absorption (Uv) detector for irradiating the eluent with ultraviolet light at a wavelength capable of producing a uniform ultraviolet absorbance response for the olefins in the sample, preferably this wavelength is equal to or less than 190 nanometers, and measuring the ultraviolet absorbance of the olefins in the eluent over the first time interval; passing the eluent through a mass detector (FID), capable of measuring the mass response to saturates and olefins in the eluent obtained over the first time interval, and capable of measuring the mass response to aromatics in the eluent obtained over the second time interval; determining the quantitative amounts of saturates, olefins and aromatics in the composition from the measurements of each detector.

In accordance with an embodiment of the invention, the said measurements generated by said ultraviolet absorption detector are stored and integrated over said first time interval to obtain an integral of absorbance and said measurements generated by said mass detector are stored and integrated over said first and second time intervals to obtain a first integral of mass and a second integral of mass wherein:

(i) the amount of aromatics expressed as weight percent of aromatics is represented by the ratio of said second integral of mass to the sum of said first and said second integral of mass;

(ii) the amount of saturates and olefins expressed as the weight percent saturates and olefins is represented by the ratio of the first integral of mass to the sum of the first and second integral of mass; and

(iii) the amount of olefins expressed as the weight percent olefins is determined from the equation: $0 = R/F (S + 0)$ where $0$ is the weight percent olefins; $(S + 0)$ is the weight percent saturates and olefins, $R$ is the ratio of said integral of absorbance to the said first integral of mass; $F$, an olefin calibration factor, is the ratio of said integral of absorbance to the first integral of mass for a known olefin;

(iv) the amount of saturates expressed as the weight percent saturates is determined by subtracting the weight percent of olefins obtained in (iii) from the weight percent of saturates and olefins obtained in (ii).

In one embodiment in accordance with the present invention, the response signals from the Uv and mass detectors are integrated in order to generate data from which a series of mathematical relationships, equations 1 to 4, can be solved to determine the weight percent of each component present.

The weight percent aromatics, A, is determined by the mathematical relationship:

$$A = \frac{\text{second integral of mass}}{\text{first integral of mass + second integral of mass}} \qquad (\text{Eq. 1})$$

where the first integral of mass and second integral of mass are the integrated signals from the mass detector over the first and second time intervals, respectively.

The weight percent saturates and olefins, B, is determined by the mathematical relationship:

$$B = \frac{\text{first integral of mass}}{\text{first integral of mass + second integral of mass}} \qquad (\text{Eq. 2})$$

6

The weight percent olefins, C, is determined by the mathematical relationship:

$$C = \frac{R}{F} (A + B) \quad (Eq. 3)$$

where R is the ratio of the integral of absorbance (the integrated signal form the Uv detector) to the first integral of mass; and F, olefin calibration factor, is the ratio of the integral of absorbance to the first integral of mass for a known olefin. The olefin calibration factor is determined by injecting a known olefin into the measurement devices and solving equation 3 for F.

Finally, the weight percent saturates, D, is determined by the mathematical relationship:

$$D = (A + B) - C \quad (Eq. 4)$$

where A, B and C are as defined above.

## DETAILED DESCRIPTION

The composition under investigation may be a feedstock for a refining process or an intermediate processed oil. The present invention could be a useful analytical tool that would, for instance, enable a refining process or processes to be adjusted as necessary to produce a product and/or intermediate product having a composition of saturates, olefins and aromatics which matches or closely matches the optimum specification for the product and/or intermediate product.

The method relies upon a chromatography column packed with a thermally stable packing material capable of separating the saturate, olefin and aromatic components of a sample of a composition. The packing normally consists essentially of an inert porous solid support that may also have distributed thereon a non-selective stationary phase. Suitable packing materials include silica, alumina, zeolites, surface modified silica, alumina and zeolites and mixtures thereof; preferred is a silica. An example of a surface modified packing material would be a silica gel stationary phase bonded with alkyl groups or with a bonded phase containing amino-cyano groups. Packed columns having the above materials are commercially available.

The stationary phase of the column is used to produce a distribution (i.e. separation) of the components of the composition which later elute from the stationary phase depending on their particular elution order at a series of times that corresponds with the components affinity for the stationary phase. To insure highly efficient separations, it is preferred that the stationary phase material have a particle size of about 3 micrometers to about 10 micrometers and a pore diameter from about 60 angstroms to 300 angstroms. Pumps may be used to provide a constant or programmed pressure throughout the chromatography column, in the range from about 100 to about 350 atmospheres. The column is loaded with the saturates, olefins and aromatics of the composition by injecting a sample of the composition onto the stationary phase. The injection volume or mass is proportional to the mass or volume of the stationary phase and when expressed in volumetric units is preferably in the range of about 1 nanoliter to about 1 microliter.

In carrying out the elution, the mobile phase is passed through the stationary phase having the components retained thereon. Depending on the components affinity for the stationary phase, the component either localizes at some point on the stationary phase to be slowly eluted off or passes through the stationary phase along with the mobile phase.

Mobile phases suitable for carrying out the elution consist of supercritical fluids selected from carbon dioxide, nitrous oxide, sulfur hexafluoride, xenon and mixtures thereof; preferred is carbon dioxide. The mobile phase is passed through the stationary phase under supercritical conditions. Preferably, in the case of carbon dioxide, these conditions consist of temperatures ranging from about 30°C to about 50°C and pressures ranging from about 100 atmospheres to about 350 atmospheres. However, when xenon is used, the temperature must be maintained below about 17°C. The flowrate of the mobile phase through the column may be controlled by installing a porous flow restrictor in front of the mass detector. Preferred flowrates are in the range of from about 0.05 cm/sec to about 0.3 cm/sec. In general, the mobile phase will have a low viscosity and a relatively low solvent strength to enable the chromatography column to effectively resolve or separate the saturates, olefins and aromatic components of the composition.

The eluent exiting the column contains the mobile phase, having passed over the stationary phase, along with the saturate, olefin and aromatic components whose concentration in the eluent varies over time. Therefore, the components are identified by their corresponding retention volume, being the time at constant flow rate after which a component is liberated from the stationary phase in the eluent. The retention volume is characteristic of each component based on its relative affinity for either the mobile or stationary phase. The saturate and olefin components are determined or considered as those components having a retention volume between 5.5 to 7.0 minutes and the aromatics are determined or considered as the component having a retention volume between 7.0 to 10.5 minutes. The overall time for the elution is less than about 11 minutes.

Once identified, the amount or concentration of these components in the eluent is measured over specific time intervals. The first time interval begins from the time the sample is injected and ends when the concentration of saturates and olefins detected in the eluent stops diminishing. The second time interval begins no sooner than the end of the first time interval and ends when the concentration of aromatics detected in the eluent stops diminishing. However, in the event where multiple time intervals exist, the end of the second time interval is determined by injecting a model aromatic compound of a known retention volume; preferred is benzene.

Detectors located at the output of the chromatograph column, monitor the level of saturate, olefin and aromatic components in the eluent exiting the column. The response from these detectors is essential in determining when the concentration of a given component detected in the eluent has stoped diminishing.

The ultraviolet absorbence (Uv) detector is known to have a response that varies greatly with the different olefin types that are contained in a composition. This holds true for a wide range of wavelengths. We have found that aliphatic olefins, for example of the type present in gasoline fractions, are shown to have ultraviolet light spectra that tend to converge at low wavelengths to give a uniform ultraviolet absorbence response. Therefore, the Uv detector may be used to monitor all of the olefins in a composition or mixture at a single wavelength. A wavelength of substantially 190 nanometers is preferred. Since the aromatic components may have differing ring numbers and substitutions and show differing ultraviolet absorbence spectra, it is not therefore possible to choose a single wavelength to determine all of the aromatic compounds. Therefore, the Uv detector is used as an olefin specific detector, since the olefins converge at the low wavelength to produce a single response.

This discovery has led to a simple and rugged chromatography analysis that can be used to produce shorter analysis times than realized before. As a result only a single chromatogrphy column is needed eliminating the need for column switching and/or back-flushing. Furthermore, the Uv detector only requires calibration with a standard olefin.

The total mass of the components eluting from the column is determined by a mass detector which may consist of a mass spectrometer or flame ionization detector (FID); preferred is the FID. The FID is constructed of a block of high thermal conductivity material, such as aluminum or brass, and is provided with a column inlet, a hydrogen inlet, an air inlet, an electrical inlet and a heater. The FID is heated to produce gases of the components in the eluent. Preferable the FID is heated to provide a temperature that is above the dew point of the gases formed in the detector. This may be accomplished by circulating a heat medium or by other means, but preferably an electrical cartridge heater fitted into a well at the base of the detector is used. The quantitative amounts of components is proportional to the number of ions formed. The response from the detector is linear over a wide range of sample concentrations and temperatures. Since the seperated components are consumed in the FID measurement, it is important to locate the Uv detector ahead of the FID.

The signals from both detectors may be amplified before being recorded and integrated.

The recorder tracing develops a profile or plot of the component distribution in the eluent as a function of time. Preferably, the recorder plot is generated from a recording potentiometer which is commercially available. The plot is in the form of time versus the concentration of the components detected in the eluent. The output of each detector is manifest by a series of signals over time separated by periods of time when no signal occurs. Increases in the response level from the base line are due to one or more of the components in the eluent. The level of the response signal for each detector before and after a response signal occurs is termed the background level and represents principally the absorbence level (Uv detector) or conductivity (ion) level (FID) of the mobile phase, also referred to as the base line.

The first step is to determine whether such increased response level is due to a component or to several components that have migrated through the chromatography column at essentially the same time. The plot will show the time intervals along the abscissa and the response level, above the base level, along the ordinate. Time intervals are in minutes and begin from a data time "0" which is right after a sample of the composition is introduced to the chromatography column. There is a time lag before the detectors can detect any deviation from the steady base line response signal. Once the ultraviolet absorbence detector detects an increase in the concentration of olefins in the eluent, a sharp increase in the response from the base line signal of the detector follows. When the concentration of olefins in the eluent stops diminishing and returns to the initial base line value for the response signal. The FID response occurs in a similar manner, however, for saturates and olefins over a first time interval and aromatics over a second time interval.

The integrator readings provide a printed record used to calculate weight percentages of the various components removed from the column based on their retention volume. The integrator may be a printing electronic integrator which produces at least 6,000 counts per minute. The integrator makes measurements on the recorded area of the chromatogram at constant time intervals, preferably about every 1/15 second and converts this data into usable information which reflects the concentration of a component in the eluent. In this manner, the integrator provides a method for calculating the weight percent of the components separated over the different time intervals. The integrated readings are suitable for processing in a computer adaptable for the automatic operation of a process.

Having described the invention, the following are examples which illustrate the various workings of it. They are not intended to limit the invention in any way.

## Example 1

Eleven gasoline samples of various grades were analyzed under the analytical method of the present invention and compared to results obtained from the standard FIA test method (ASTM D-1319). To control the evaporation of the lower boiling point components in each sample, the samples were refrigerated prior to their use. The supercritical fluid chromatographic column was fitted to an injector which was water cooled. The column was inserted directly into one of the ports of the injector without the need for a connection piece. Portions of the samples were injected into the chromatograph either through total loop injection or at split time intervals which involved rapid rotation (0.02 sec) of a 60 nanoliter injection loop. The column had dimensions of 1 mm I.D., 1/16 inch O.D. and 50 cm in length The stationary phase was a silica packing material having a particle size of 10 micrometers. The supercritical fluid mobile phase was carbon dioxide and was maintained at a flow rate of 16 cc/min, measured at the exit of the FID detector after passing through the stationary phase. The column was operated at 40°C and at a constant pressure of 200 ATM. The ultraviolet absorbence (Uv) detector was designed for operation with packed column supercritical fluid chromatography. The flow-through cell of the detector held a volume of 0.25 ml and had a path length of 2 mm and was water cooled. The upper limit of the linear response of the Uv detector was sometimes exceeded if the olefin concentration in the eluent from the column varied significantly. Therefore, to ensure that the olefin Uv response fell within the detector's linear response range of 1.4 absorbance units, each sample was re-run at a lower split time interval when the response signal exceeded 1.4 absorbance units. The FID required flow rates of 380 cc/min air, 30 cc/min $H_2$ and operated at a temperature of 325°C. The Uv detector was placed ahead of the flow restrictor but outside of the oven with the fluid lines emerging from and returning to the oven after transversing the UV detector. The Uv detector's response to olefins was calibrated with a standard olefin. The data from each detector was recorded and integrated by computer. The analysis time was 11 minutes.

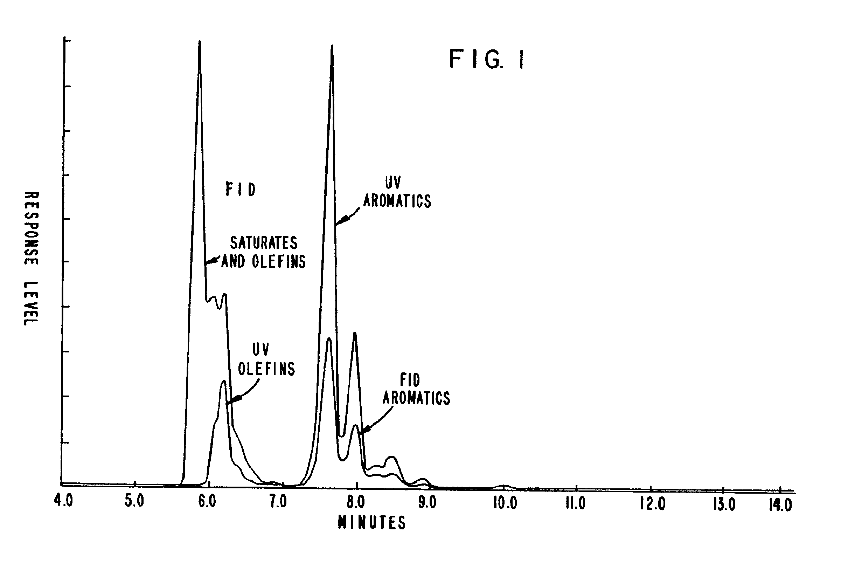

According to the FID response shown in Figure 1 a clean seperation of olefins and saturates from aromatics was acheivable for each gasoline sample. However, separation between olefins and saturates was not detected. The Uv detector response shows that the olefins have an elution time that is at the tailing edge of the elution time for the saturates.

The results are in terms of weight percent saturates, olefins and aromatics measured in the gasoline samples by the present analytical method and in terms of volume percent by the FIA method are compared in Table I.

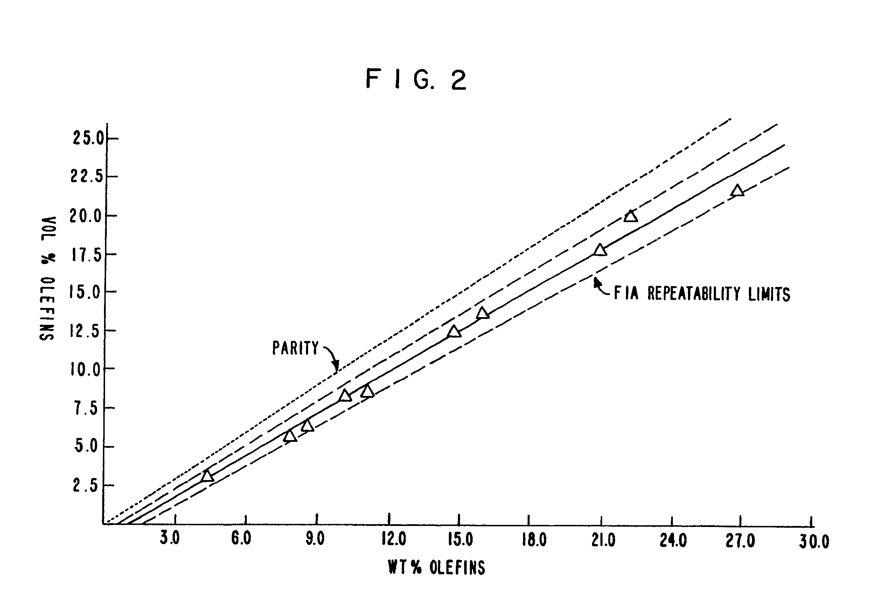

Figure 2 shows that the results correlate with and are well within the repeatability limits established for the standard FIA method. This demonstrates that differences in olefin levels, as measured by the FIA method, are measured equally as well by the present analytical method. There exists a bias towards lower olefin results by the FIA method. The reason is that the densities of the various hydrocarbon types within the gasoline sample can be expected to differ. This leads to slightly different levels of results in view of the fact that FIA measures components in volume percent and the new method in weight percent.

## Example 2

The method of Example 1 was carried out using a specific RR-2 reference gasoline sample. The results in terms of wt.% saturates, olefins and aromatics are listed in Table II. The results show a two sigma repeatability of 0.75 at an olefin level of 12 wt.% which compares to a value of 1.3% for the standard FIA method. This demonstrates that the short term precision for measuring olefins is better than that reported using the FIA method.

## Example 3

The method of Example 1 was carried out on a simulated gasoline blend having a 63.9 wt.% petroleum ether, 9.4 wt.% Blend II and 26.7 wt.% Aromatics (see Table III). The results showed 9.6 wt.% olefins 28 wt.% aromatics and 62.6 wt.% saturates. This shows that the present method is accurate, being capable of measuring a true level of saturates, olefins and aromatics.

## Example 4

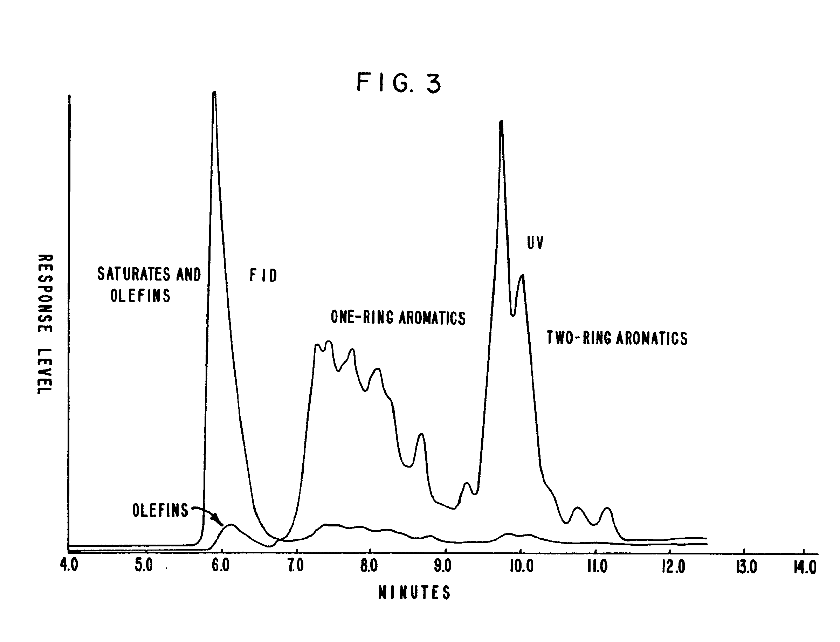

The method of Example 1 was carried out on a jet fuel sample, the results of which are illustrated in Figure 3. The separation of one and two ring aromatic components is quite apparent from the Uv detector. Further analysis of the aromatic components can readily be quantified by the FID detector. The FIA method response for the olefins was 2.6% versus 0.4% by the present method. However, the olefin value from the FIA method is uncertain. This demonstrates that the present method is applicable to the analysis od jet fuels.

Although particular embodiments of the invention have been shown and described, it is to be understood that the scope of the invention is not limited to these embodiments, since modifications can be made by one skilled in the art.

TABLE I

## A COMPARATIVE DISTRIBUTION OF HYDROCARBON COMPOUNDS
## IN COMMERCIAL GASOLINES BY FIA AND SFC ANALYSIS METHODS

| GASOLINE | FIA (VOL %) | | | SFC (WT%) | | |
|---|---|---|---|---|---|---|
| | Saturates | Olefins | Aromatics | Saturates | Olefins | Aromatics |
| REG-UL | 53.9 | 8.4 | 37.7 | 43.3 | 10.2 | 46.5 |
| PREM-UL | 56.7 | 5.9 | 37.3 | 46.7 | 7.9 | 45.4 |
| REG-LD | 58.0 | 8.7 | 33.3 | 45.8 | 11.2 | 43.0 |
| REG-UL | 63.6 | 12.6 | 23.8 | 56.9 | 14.8 | 28.3 |
| SUPER-UL | 52.5 | 6.5 | 41.1 | 42.5 | 8.6 | 48.9 |
| REG-UL | 47.6 | 20.1 | 32.3 | 45.1 | 22.2 | 32.7 |
| PLUS-UL | 37.3 | 21.9 | 40.8 | 36.4 | 26.8 | 36.8 |
| SUPER-UL | 42.5 | 13.8 | 41.1 | 39.4 | 16.0 | 44.6 |
| REG-UL | 58.4 | 17.9 | 23.7 | 53.1 | 20.9 | 26.0 |
| PLUS-UL | 55.9 | 3.4 | 40.7 | 46.7 | 4.4 | 48.9 |
| SUPER-UL | 58.0 | 8.7 | 33.3 | 50.4 | 10.1 | 39.5 |

REG - Regular
PREM - Premium
UL - Unleaded
LD - Leaded

## TABLE II

### RR-2 GASOLINE ANALYSIS BY SFC

### CONCENTRATIONS, WT%

|  | Saturates | Olefins | Aromatics |
|---|---|---|---|
|  | 56.51 | 11.55 | 31.94 |
|  | 56.16 | 12.54 | 31.30 |
|  | 57.46 | 12.18 | 30.36 |
|  | 58.08 | 12.50 | 29.42 |
|  | 59.00 | 12.70 | 28.30 |
|  | 58.80 | 12.40 | 28.80 |
|  | 58.86 | 12.48 | 28.66 |
| Average Value (X) | 57.84 | 12.33 | 29.83 |
| Standard Deviation ($\sigma$) | 1.07 | 0.352 | 1.30 |
| Standard Deviation (%$\sigma$) | 1.86 | 2.86 | 4.36 |

## TABLE III

### COMPOSITION OF SYNTHETIC HYDROCARBON
### BLENDS PREPARED FOR THE SFC SYSTEM

| OLEFINS BLEND I | OLEFINS BLEND II |
|---|---|
| 1-heptene | 2-methyl-2-butene |
| 3-heptene | 4-methyl-1-pentene |
| 2-ethyl-1-hexane | 2-methyl-2-pentene |
| diisobutylene | cis-4-methyl-2-hexane |
| dipentene | trans-2-heptene |
| | 2,2,4-trimethyl-2-pentene |
| | 1-octene |
| | 2-methyl-2,4-pentadiene |

**AROMATICS**

1,2,4-trimethylbenzene
xylenes
toluene

| GASOLINE BLEND | WT% |
|---|---|
| Petroleum ether | 63.9 |
| Olefin Blend II | 9.4 |
| Aromatic Blend | 26.7 |

**Claims**

1. A method for determining the quantitative amount of saturates, olefins and aromatics in a composition comprising :

    (a) contacting a chromatographic stationary phase with a sample of the composition for a time sufficient to load said saturates, olefins and aromatics onto said stationary phase;

    (b) separating said saturates, olefins and aromatics, loaded on said stationary phase, by passing a mobile phase, under super critical conditions, through said stationary phase in a sufficient amount and for a time sufficient to obtain an eluent wherein the amount of saturates, olefins and aromatics in said eluent varies over time such that the amount of saturates and olefins in said eluent obtained over a first time interval is substantially higher than over a second time interval, being a time interval at least after said first time interval and the amount of aromatics in said eluent obtained over said second time interval is substantially higher than over said first time interval;

    (c) passing said eluent through an ultraviolet absorption detector for irradiating said eluent with ultraviolet light at a wavelength capable of producing a uniform ultraviolet absorbance response for said olefins and measuring said ultraviolet absorbance response over said first time interval;

    (d) passing said eluent through a mass detector capable of producing a mass response to saturates and olefins in said eluent obtained over said first time interval and capable of producing a mass response to said aromatics in said eluent obtained over said second time interval and measuring said mass response over said first time interval and said second time interval;

    (e) determining the quantitative amount of saturates, olefins and aromatics from said measurements obtained in steps (c) and (d).

2. The method of claim 1 wherein said measurements generated by said ultraviolet absorption detector are stored and integrated over said first time interval to obtain an integral of absorbance and said measurements generated by said mass detector are stored and integrated over said first and second time intervals to obtain a first integral of mass and a second integral of mass wherein:

    (i) the amount of aromatics expressed as weight percent of aromatics is represented by the ratio of said second integral of mass to the sum of said first and said second integral of mass;

    (ii) the amount of saturates and olefins expressed as the weight percent saturates and olefins is represented by the ratio of the first integral of mass to the sum of the first and second integral of mass; and

    (iii) the amount of olefins expressed as the weight percent olefins is determined from the equation: $0 = R/F (S + 0)$ where $0$ is the weight percent olefins; $(S + 0)$ is the weight percent saturates and olefins, $R$ is the ratio of said integral of absorbance to the said first integral of mass; $F$, an olefin calibration factor, is the ratio of said integral of absorbance to the first integral of mass for a known olefin;

    (iv) the amount of saturates expressed as the weight percent saturates is determined by subtracting the weight percent of olefins obtained in (iii) from the weight percent of saturates and olefins obtained in (ii).

3. The method of claim 1 or claim 2 wherein the wavelength capable of producing a uniform ultraviolet absorbance response for olefins is equal to or less than 190 nanometers.

4. The method of any one of claims 1 to 3 wherein the amount of aromatics in said eluent over said first time interval is negligible.

5. The method of any one of claims 1 to 4 wherein the composition is selected from hydrocarbons in the naphtha to middle distillate boiling range, and/or $C_5$ to $C_{20}$ hydrocarbons, and/or wherein the composition is in the gasoline boiling range.

6. The method of any one of claims 1 to 5 wherein the stationary phase is selected from the group consisting of silica, alumina, zeolites, surface chemical modified silica, alumina and zeolites and any mixture thereof.

7. The method of any one of claims 1 to 6 wherein the mobile phase is selected from the group consisting of carbon dioxide, nitrous oxide, sulfur hexafluoride, xenon and any mixture thereof.

8.  The method of any one of claims 1 to 7 wherein said supercritical conditions consist of temperatures of from about 30°C to about 50°C and pressures of from about 100 atmosphere to about 350 atmosphere.

9.  The method of any one of claims 1 to 8 wherein said mass detector is either a flame ionization detector or a mass spectrometer detector.

10. The method of any one of claims 1 to 9 wherein said stationary phase has a pore diameter ranging from about 60 angstroms (6 nm) to about 300 angstroms (30 nm) and a particle size ranging from about 3 micrometers to about 10 micrometers.

FIG. 1

16

# F I G. 2

FIG. 3